# EUROPEAN PATENT APPLICATION

(11) **EP 2 502 620 A1**
(43) Date of publication of application: **26.09.2012**
(21) Application number: 11159622.7
(22) Date of filing: 24.03.2011
(51) Int. Cl.: A61K 9/20, A61K 31/445

(54) **Solid pharmaceutical composition comprising donepezil**

(71) Applicant: Krka Tovarna Zdravil, D.D., Novo Mesto, 8501 Novo Mesto (SI)
(72) Inventor: Kroselj, Vesna, 8000 Novo Mesto (SI); Zakelj, Mojca Segula, 1000 Ljubljana (SI); Skube, Maja Kincl, 8000 Novo Mesto (SI); Ritlop, Gregor, 1000 Ljubljana (SI)
(74) Representative: UEXKÜLL & STOLBERG

(57) **Abstract**

The present invention relates to a solid pharmaceutical composition comprising (a) at least one drug selected from donepezil or a pharmaceutically acceptable salt or solvate thereof and (b) at least one matrix former which is selected from the group consisting of cellulose derivatives, starch derivatives and mixtures thereof, wherein the matrix former has a viscosity of 80 to 120,000 mPa·s, determined for a 2 % (w/v) aqueous solution at 20°C, as well as a process for its preparation.

## Description

The present invention relates to a solid pharmaceutical composition comprising donepezil or a pharmaceutically acceptable salt or solvate thereof as well as to a process for preparing such a composition.

Donepezil, i.e. (±)-2,3-dihydro-5,6-dimethoxy-2-[[1-(phenylmethyl)-4-piperidinyl]methyl]-1*H*-inden-1-one, is represented by chemical formula (I)

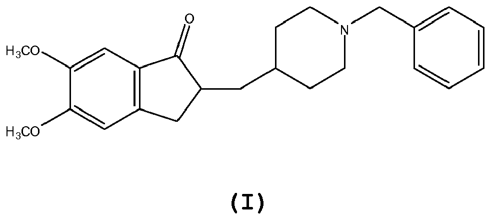

Donepezil is known as a reversible inhibitor of acetylcholinesterase. Donepezil and its salts are used for the treatment of a variety of disorders including dementia and attention deficit disorder, particularly for the symptomatic treatment of Alzheimer's disease. It is currently marketed as 5 mg, 10 mg and 23 mg film-coated tablets ARICEPT^{®} as well as 5 mg and 10 mg orodispersible tablets ARICEPT^{®} ODT.

Donepezil is known in several polymorphic forms and WO 97/46527 describes a method for the preparation of the polymorphic forms I to V as well as the amorphous form of donepezil hydrochloride.

EP 1 086 706 describes the preparation of a composition comprising donepezil and an organic acid selected from tosyllic acid, mesyllic acid, benzoic acid, salicylic acid, tartaric acid and citric acid. It is reported that the use of such an organic acid results in an increase of light and heat stability of donepezil compared to the use of the donepezil hydrochloride salt form which yields a relatively high amount of impurities upon storage.

EP 1 378 238 describes the preparation of donepezil hydrochloride in stable amorphous form as well as a pharmaceutical composition for the treatment of dementia or Alzheimer's disease in which the active therapeutical agent is donepezil hydrochloride in an amorphous state.

EP 1 811 957 discloses a pharmaceutical composition comprising donepezil hydrochloride in which the donepezil hydrochloride is highly stable against polymorphic conversion and retains its polymorphic form throughout the shelf life of the composition.

Sustained release preparations of donepezil are of great interest since such formulations would allow the number of drug administrations to be reduced while providing the same or better therapeutic effects improving patient compliance and minimizing side effects. WO 2006/070930 describes sustained release formulations comprising an anti-dementia drug, a high molecular weight basic substance and a high molecular weight acidic substance, like an enteric polymer, for stabilizing the anti-dementia drug. It is described that addition of the high molecular weight acidic substance, such as methacrylic acid-ethyl acrylate copolymer, effectively prevents or inhibits the formation of degradation products of the anti-dementia drug which are produced by contact of the ant-dementia drug with the high molecular weight basic substance, i.e. the sustained-release base. The high molecular weight acidic substance can also be combined with a low molecular weight acidic substance and an anti-oxidant.

Likewise, the commercially available ARICEPT^{®} 23 mg tablets include donepezil hydrochloride, ethylcellulose (i.e. a high molecular weight basic substance), hydroxypropyl cellulose, lactose monohydrate, magnesium stearate and methacrylic acid copolymer, Type C (i.e. a high molecular weight acidic substance). The film coating includes ferric oxide, hypromellose 2910, polyethylene glycol 8000, talc and titanium dioxide.

WO 2005/065645 is directed to pharmaceutical compositions of amorphous donepezil. Several dosage forms having different release properties are described and sustained release dosage forms are reported to comprise a release-retarding material selected from acrylic polymers, alkylcelluloses, shellac, zein, hydrogenated vegetable oil, hydrogenated castor oil or combinations thereof. No particular sustained-release formulation is however disclosed in this reference.

Apart from achieving a desired specific release of active pharmaceutical ingredient by a sustained-release formulation, special care must be taken that the stability of the drug is not affected. Like many other anti-dementia drugs, donepezil has an amino group which is in general a highly reactive functional group having nucleophilic properties that can easily result in the formation of degradation products when contacting carbonyl groups, peroxides, oxygen or the like. Special care should also be taken since possible sustained-release excipients and other additives are not included in conventional, fast-dissolving tablets and their effect on donepezil stability is not well described yet.

Thus, there is still a need for a simple solid pharmaceutical composition of donepezil or a salt or solvate thereof having a satisfactory sustained drug release profile and having excellent stability of donepezil. In particular, it is desired that chemical and polymorphic conversions of the active ingredient during preparation and storage of the pharmaceutical composition are avoided. Moreover, the composition should not require the use of expensive excipients and should be obtainable by a simple, cost efficient and quick process.

These objects are surprisingly solved by the solid pharmaceutical composition according claim 1 to 13. The invention also relates to the processes according to claims 14 and 15.

In a first aspect, the invention relates to a solid pharmaceutical composition which comprises
(a) at least one drug selected from donepezil or a pharmaceutically acceptable salt or solvate thereof, and
(b) at least one matrix former which is selected from the group consisting of cellulose derivatives, starch derivatives and mixtures thereof.

Preferably, the matrix former has a viscosity of 80 to 120,000 mPa·s, determined for a 2 % (w/v) aqueous solution at 20°C.

Thus, the invention particularly relates to a solid pharmaceutical composition comprising
(a) at least one drug selected from donepezil or a pharmaceutically acceptable salt or solvate thereof, and
(b) at least one matrix former which is selected from the group consisting of cellulose derivatives, starch derivatives and mixtures thereof, wherein the matrix former has a viscosity of 80 to 120,000 mPa·s, determined for a 2 % (w/v) aqueous solution at 20°C.

The component (a) of the composition according to the invention is a drug selected from donepezil or a pharmaceutical acceptable salt or solvate thereof. Examples of useful salts are the addition salts with an inorganic or organic acid. Examples of useful inorganic acids include hydrochloric, hydrobromic, phosphoric and sulfuric acid. Examples of suitable solvents for preparing solvates of donepezil include water, ethanol and isopropanol. Preferably, the drug (a) is donepezil hydrochloride.

The drug (a) can for example be prepared according to a process as decribed in EP 1 378 238, EP 1 513 528 and EP 1 811 957.

The drug (a) can be in a non-crystalline, i.e. amorphous, form or a crystalline form including any polymorphic or pseudopolymorphic forms such as solvates like hydrates.

Preferably, the drug is donepezil hydrochloride in one of its various polymorphic forms, in particular form I or form IV. These polymorphic forms of donepezil hydrochloride as well as the preparation thereof are disclosed in WO 97/46527 the contents of which are incorporated herein by reference. The compositions of the present invention thus preferably contain polymorph I and/or polymorph IV of donepezil hydrochloride in the form of a hydrate. A composition is particularly preferred wherein drug (a) is donepezil hydrochloride of polymorphic form I and in particular the monohydrate of polymorphic form I. This form has been found to be very stable against undesired changes to other polymorphic forms of the active ingredient.

Donepezil hydrochloride hydrate of form I or form IV is preferably prepared by suspending donepezil hydrochloride in a solvent. Alternatively donepezil hydrochloride can be prepared from donepezil base and hydrochloric acid. Preferably, a mixture of methanol and water is used as the solvent. Optionally other alcohols, such as ethanol or isopropanol, or mixtures of alcohols with water can be used. The formation of donepezil hydrochloride hydrate form I or form IV depends upon the water content in the solvent mixture. The suspension is heated until the donepezil hydrochloride has completely dissolved in the solvent. Optionally this solution can be filtered through 1 PM filtration cartridge. The solution of donepezil hydrochloride is then cooled to approximately 40°C. Donepezil hydrochloride hydrate of form I or form IV is precipitated from this solution by the addition of isopropyl ether, isopropyl acetate, ethyl acetate, butyl acetate, isobutyl methyl ketone, tert.-butyl methyl ether or heptane.

Hence, a crystalline form of the drug and most preferably crystalline donepezil hydrochloride like donepezil hydrochloride polymorphic form I is used.

The particle size of the drug (a) is d90 < 300 µm, more preferably < 250 µm, most preferably < 200 µm, measured by methods known in the art, such as laser diffraction method on Malvern Mastersizer.

It is preferred that the amount of drug (a) is 1 to 60 wt.-%, preferably 1 to 50 wt.-%, such as 1 to 40 wt.-%, and most preferably 1 to 30 wt.-%, such as 5 to 20 wt. %, based on the total weight of the pharmaceutical composition. It is also preferred that a single dosage form of the pharmaceutical composition according to this invention comprises 1 to 50 mg, preferably 2 to 40 mg and most preferably 5 to 30 mg of drug (a). In another embodiment, the solid pharmaceutical composition according to the invention comprises donepezil or a pharamaceutically acceptable salt of donepezil in an amount of 1 mg to 60 mg, preferably 8 mg to 36 mg or 10 mg to 30 mg, calculated as free base of donepezil.

The component (b) of the composition is a matrix former. As used herein, the term "matrix former" denotes a hydrophilic polymer that upon contact with an aqueous medium is capable of forming a hydrogel, i.e. a swollen, water-containing network of polymer chains that are water-insoluble. Thus, the cellulose or starch derivative polymer is capable of forming a matrix, which ensures, inter alia, the sustained-release of drug (a). Release of the drug in physiological medium may take place both by means of diffusion and by means of erosion of the matrix. In particular, the matrix allows sustained-release of the drug over at least 6 hours, i.e. about 40 to about 80 % of drug (a) are dissolved within 6 hours in a standard *in vitro* dissolution test.

Hence, the solid pharmaceutical composition is a matrix type sustained-release preparation having a matrix in which the drug is uniformly distributed. As used herein, the term "matrix preparation" refers to preparations as for example described in J.R. Cardinal, Drug release from matrix devices, in: J.M. Anderson, S.W. Kim (Eds.), Recent Advances in Drug Delivery Systems, Plenum Press, New York, 1984, pp. 229-248. The composition suitably has the form of a tablet or granules, and may be film coated. Coated sustained-release compositions can include those in which a coating comprising a sustained-release base is applied to a drug containing tablet core or granule core, or those in which a coating comprising a sustained-release base is applied to a layer containing the drug which is in turn applied on a core particle consisting of crystalline cellulose or sucrose, i.e. a so-called nonpareil. The sustained-release characteristics can also controlled by the presence of multiple layers of drug containing coating and sustained-release base containing coating.

The component (b) is a cellulose derivative or starch derivative or a mixture thereof, and preferably is a cellulose derivative. As used herein, the term "cellulose derivative" denotes cellulose ethers and/or cellulose esters. Preferably, the cellulose derivative (b) is a cellulose polymer wherein the hydroxyl groups of cellulose are at least partially replaced by substituents -OR with R being selected from the group consisting of alkyl, hydroxyalkyl, -C(O)alkyl, -C(O)aryl and mixtures thereof, wherein said alkyl or aryl can be unsubstituted or optionally substituted. Said optional substituents on said alkyl or aryl groups can be selected from the group consisting of halo, -CF₃, -CN, -COOH, and mixtures thereof. A preferred optional substituent is -COOH. As used in these definitions, "alkyl" means a straight or branched aliphatic carbon chain preferably comprising 1 to about 6 carbon atoms. Further, as used in the above definitions "aryl" means an aromatic carbon ring system having from about 5 to about 14 carbon ring atoms and preferably is phenyl.

In one embodiment, the cellulose derivative is a cellulose polymer in which at least a part of the hydrogen atoms of the hydroxyl groups of cellulose has been replaced by hydroxyalkoxy groups, such as hydroxyethoxy or hydroxypropoxy groups. In particular, the cellulose compound (b) comprises hydroxypropoxy groups. Instead of or in addition to hydroxyalkoxy groups, the cellulose compound (b) may also comprise alkoxy groups, like methoxy or ethoxy.

It is preferred that the hydroxyalkoxy content of cellulose derivative (b), i.e. the degree of hydroxyalkoxy substitution, ranges from 0 % to 80 %, preferably from 0% to 30 %, most preferably from 7 % to 12%, as determined in accordance with a gas chromatography method like that described in Ph.Eur. (Monograph "Hypromellose", 7th Ed., 2011, (7.0)).

Furthermore, it is preferred that the alkoxy content of cellulose derivative (b), i.e. the degree of alkoxy substitution, ranges from 0 % to 40 %, preferably from 15 % to 35 %, most preferably from 19% to 24 %, as determined in accordance with a gas chromatography method like that described in Ph.Eur. (Monograph "Hypromellose", 7th Ed., 2011, (7.0)).

In particular, the cellulose derivative (b) can be selected from, but is not limited to the group consisting of ethylcellulose, methylcellulose, cellulose acetate, cellulose acetate phthalate, hydroxymethyl cellulose, hydroxyethyl cellulose, hydroxypropyl cellulose (HPC), hydroxypropyl methylcellulose (HPMC) and mixtures thereof.

It is particularly preferred that the cellulose derivative (b) is hydroxypropyl methylcellulose (HPMC). HPMCs are commercially available polymers known to the person skilled in the art and customarily used in the field of medicament formulation. It is to be noted that these polymers are marketed for example under the trade marks Methocel^{™} and Metolose^{™}.

The starch derivative can be starch or chemically and/or mechanically modified starch like pregalitinized starch. Preferably, pregelatinized starch is used as starch derivative.

It has surprisingly been found that a desired sustained-release profile of donepezile and a desired stability of donepezil can be achieved by use of a cellulose and/or starch derivative (b) having a specific viscosity.

As noted above, the matrix former has preferably a viscosity of 80 to 120,000 mPa·s, determined for a 2 % (w/v) aqueous solution at 20°C. In another preferred embodiment, the matrix former, and in particular the hydroxyalkyl alkylcellulose like HPMC, has a viscosity of 80 to 120 mPa·s or a viscosity of 3,000 to 5,600 mPa·s or a viscosity of 11,250 to 21,000 mPa·s or a viscosity of 80,000 to 120,000 mPa·s, as measured for a 2 % (w/v) aqueous solution at 20°C. As used herein, viscosity may in general be determined using a rotational viscometer such as the Brookfield model LVF viscometer calibrated against standard oils. Alternatively, and in particular if the matrix former is hydroxypropyl methylcellulose, then viscosity may preferably be determined in accordance with Ph.Eur. (Monograph "Hypromellose", 7th Ed., 2011, (7.0)).

Hence, it is particular preferred to use hydroxypropyl methylcellulose having a methoxy content of 19 to 24 %, a hydroxypropoxy content of 7 to 12 % and a viscosity of 80 to 120 mPa·s or 3.000 to 5.600 mPa·s, determined for a 2 % (w/v) aqueous solution at 20°C.

The bulk density of matrix former (b) may also have an effect on the properties of the resulting pharmaceutical composition and the used manufacturing procedure. Typically, the bulk density of the matrix former like HPMC is below 0.5 g/cm³, preferably below 0.4 g/cm³ and more preferably below 0.25 g/cm³, as determined by methods known in the art like using a volumeter in accordance with Ph.Eur. - 2.9.34 (7th Ed., 2011, (7.0)).

In a particularly preferred embodiment, direct compression grades of HPMC are used as matrix former (b).

Moreover, it is particularly preferred that the particle size of matrix former (b) like HPMC is such that more than 99 wt.-% of the particles pass through a 40 mesh sieve and more than 90 wt.-% of the particles pass through a 100 mesh sieve.

The average particle size of matrix former (b) like HPMC ranges from about 50 µm to about 200 µm, preferably from about 80 µm to about 150 µm. The average particle size can for example be determined by laser diffraction analysis using an apparatus having a He-Ne laser providing light with a wavelength of 0.633 nm and equipped with a dry powder feeder using Mie light scattering theory, such as a Malvern Mastersizer 2000 equipped with Scirocco dispersion cell.

The composition preferably comprises 10 to 60 wt.-%, more preferably 15 to 50 wt.-% and most preferably 25 to 35 wt.-% of matrix former (b), based on the total weight of the pharmaceutical composition.

In addition to the matrix former (b), the pharmaceutical composition according to the invention can comprise an additional matrix former (c). Said additional matrix former (c) may be selected form the group consisting of cross-linked acrylic acid polymers and mixtures thereof. Preferably, the additional matrix former (c) is a cross-linked acrylic acid polymer, i.e. an acrylic acid polymer in which one polymer chain is linked via a bond, in particular a covalent bond, to another polymer chain. Preferably, the acrylic acid polymer (c) is cross-linked with allyl or vinyl compounds. It is particular preferred that the acrylic acid polymer (c) is cross-linked with allyl sucrose, allyl pentaerythriol, allyl propylene and/or divinyl glycol. More preferably, the acrylic acid polymer (c) is a carbomer and most preferably a carbomer cross-linked with allyl pentaerythriol.

In one embodiment, the composition comprises a cellulose compound (b) which is hydroxypropyl methylcellulose and an acrylic acid polymer (c) which is a carbomer, like a carbomer cross-linked with allyl pentaerythriol.

In a second aspect, the invention relates to a solid pharmaceutical composition which comprises
(A) granules comprising (i) at least one drug selected from donepezil or a pharmaceutically acceptable salt or solvate thereof, and (ii) at least one matrix former, and
(B) an extragranular matrix phase comprising (iii) at least one matrix former.

It has surprisingly been found that it is possible to prepare a sustained-release dosage form which has very good stability of donepezil by providing a composition comprising granules (A) as inner phase and an extragranular phase (B). The granules (A) comprise the drug (i) and a matrix former (ii), wherein the amount of (ii) in granules (A) is only a part of the complete amount of matrix former used in the final pharmaceutical composition.

As used herein, the term "extragranular matrix phase" denotes a matrix phase which is characterized in that the granules (A) are dispersed in such matrix phase. In particular, the extragranular matrix phase is not in the form of a coating.

In one embodiment, the average particle size of granules (A) ranges from about 10 µm to about 1000 µm, preferably from about 50 µm to about 500 µm. In particular, the average particle size can be determined by laser diffraction analysis.

In order to achieve the desired sustained-release of drug (i), it is preferred that extragranular matrix phase (B) shows swelling properties such that the amount of water penetrating into extragranular phase (B) in the upper GI tract allows sufficient absorption of drug (i) in the colon. In particular, the swelling capacity of the composition according to the invention is such that the swelling ratio [(D₀ - D_{obs}) / D₀], with Do being the initial diameter of a sample tablet and D_{obs} being the diameter of the portion of the sample tablet not being swelled after 2 hours, is more than 0.4, preferably more than 0.5, more preferably more than 0.6 such as more than 0.7, when measuring a round 8 mm tablet in 250 ml of 0.05M phosphate buffer solution pH 6.8 at a paddle speed of 50 rpm.

Furthermore, it is preferred that extragranular matrix phase (B) is obtainable by compressing matrix former (iii) and optionally further excipients at a compression pressure ranging from about 2 to about 60 kN, preferably less than 30 kN. Preferably, the hardness of extragranular phase (B) is about 50 to about 300 N, such as more than 80 N.

The matrix former (ii) and/or the matrix former (iii) of the composition according to the second aspect of the invention are preferably a matrix former (b) and/or (c) as described above in connection with the first aspect of the invention. Accordingly, all definitions, including the definition of preferred embodiments, given above for matrix former (b) and/or (c) shall independently also apply to matrix formers (ii) and/or (iii) according to the second aspect.

In one embodiment, matrix former (ii) and/or the matrix former (iii) of the composition according to the second aspect of the invention can independently be selected from the group consisting of cellulose derivatives, such as, for example, hydroxypropyl methylcellulose, carboxymethylcellulose, hydroxylpropylcellulose, hydroxymethylcellulose, hydroxyethylcellulose, hydroxypropyl ethylcellulose, methylcellulose, ethylcellulose, carboxyethylcellulose, carboxymethyl hydroxyethylcellulose, or cellulose acetate, polyvinylpyrrolidone, polyacrylates, polymethacrylates, polyethylene glycol, polyethylene oxide, carrageenans, carbomer, hitosan, xantan, locus bean gum, gelan, hydroxypropyl starch, derivatives of alginic acid, guar gum, gum acacia, dextran and mixtures thereof.

In another embodiment, the matrix former (ii) and/or the matrix former (iii) of the composition according to the second aspect of the invention can independently be selected from the group consisting cellulose derivatives, such as, for example, hydroxypropyl methyl cellulose, carboxymethylcellulose, hydroxypropyl cellulose, methylcellulose, ethylcellulose, or cellulose acetate, polyethylene glycol, polyethylene oxide, carrageenans and mixtures thereof.

In a further embodiment, the matrix former (ii) and/or the matrix former (iii) are independently selected from cellulose compounds (b) and cross-linked acrylic acid polymers (c) as defined above in connection with the composition according to the first aspect of the invention.

In a preferred embodiment, the matrix former (ii) and/or the matrix former (iii) are independently selected from the group consisting of hydroxypropyl methylcellulose, carbomer and mixtures thereof.

It is particularly preferred that the matrix former (ii) is hydroxypropyl methylcellulose and the matrix former (iii) is hydroxypropyl methylcellulose, carbomer or a mixture of hydroxypropyl methylcellulose and carbomer.

The composition according to the second aspect of the invention can comprise about 5 to 95 wt.-%, preferably 10 to 90 wt.-%, more preferably 20 to 80 wt.-% and most preferably 50 to 70 wt.-% of matrix former (ii) and (iii), based on the total weight of the pharmaceutical composition.

Typically, the weight ratio of matrix former (ii) to matrix former (iii) ranges from about 40:1 to 1:20, preferably about 10:1 to 1:2, more preferably about 5:1 to 1:1.5.

It has surprisingly been found that by selection of the above components of the composition according to the first aspect of the invention and according to the second aspect of the invention a stable pharmaceutical composition showing a suitable sustained drug release profile and having excellent stability of donepezil can be prepared by using a very simple and fast process. In particular, the pharmaceutical composition according to the does not require the use of organic acids selected from tosyllic acid, mesyllic acid, benzoic acid, salicylic acid, tartaric acid and citric acid for stabilizing donepezil. In fact, both chemical and polymorphic conversions are avoided upon preparation and storage of the pharmaceutical compositions according to the invention.

The solid pharmaceutical composition according to the first aspect to the invention and according to the second aspect of the invention is preferably characterized by an *in vitro* release profile wherein on average about 15 to about 55 % of drug (a) are dissolved within 2 hours, about 40 to about 80 % of drug (a) are dissolved within 6 hours and more than 80 % of drug (a) are dissolved within 20 hours after placement of the composition in a standard dissolution test. A "standard dissolution test" as used herein is a test conducted according to USP 33-28 NF second supplement, General chapter <711>, using Apparatus 1 described therein at a rotation speed of 100 rpm and a dissolution medium of 0.1 M HCl, pH 6.8 at 37 ± 0.5°C.

The solid pharmaceutical composition according to the invention is further preferably characterized by an *in vivo* absorption of drug (a) following a single dose oral administration to a human patient so that a blood plasma level profile is provided having a dosage-corrected Cₘₐₓ of 0.9 to 2.0 ng/mL·mg. The dosage-corrrected Cₘₐₓ refers to Cₘₐₓ divided by the number of milligrams of donepezil or the pharmaceutically acceptable salt thereof in the formulation. In one embodiment, the Cₘₐₓ occurs at a Tₘₐₓ of 4.0 hours to 10.0 hours. Tₘₐₓ refers to the time after dosing at which the maximum blood plasma concentration occurs. In another embodiment, the pharmaceutical composition has an AUC of 950 to 2300 ng·hr/mL.

In addition to components (a) and (b) or (a) to (c) of the composition according to the fist aspect of the invention or in addition to components (i) to (iii) according to the second aspect of the invention, the composition can comprise at least one other excipient selected from the group consisting of diluents, gel strength regulators, binders, stabilizers, glidants, lubricants, colorants and mixtures thereof.

Examples of diluents useful in the composition according to the invention include, but are not limited to microcrystalline cellulose, powdered cellulose, lactose such as anhydrous lactose or preferably lactose monohydrate, compressible sugar, fructose, dextrates, sugars like mannitol, sorbitol, lactitol, saccharose or a mixture thereof, siliconised microcrystalline cellulose, calcium hydrogen phosphate, calcium carbonate, calcium lactate and mixtures of thereof. Preferably, the excipients include at least one diluent selected from microcrystalline cellulose and lactose monohydrate.

The pharmaceutical composition according to the invention can also comprise a gel strength regulator. Such a gel strength regulator may enhance the resistance of the matrix of the composition to mechanical stress and dose dumping. Non-limiting examples of suitable gel strength regulators include polyethylene glycols and polyols. Preferably, polyethylene glycols of high molecular weight, such as for example PEG 4000, PEG 6000 and PEG 8000, and/or polyols, like sorbitol, manitol and xylitol, can be used. Most preferably, PEG 6000 and sorbitol are used as gel strength regulators. It is preferred that the pharmaceutical composition of the invention comprises drug (a), matrix former (b) and gel strength regulator.

Examples of binders useful in the composition according to the invention include, but are not limited to polyvinylpyrrolidone (povidone), cellulose derivatives, such as for example hydroxypropyl cellulose and hydroxypropyl methylcellulose, gelatine and the like.

Stabilizers such as pH modifiers and antioxidants can also be contained in the composition. For example, sodium or potassium acetate can be used as pH modifiers and butylhydroxyanisole (BHA), butylhydroxatoluene (BHT), propyl gallate, ascorbic acid, EDTA and the like can be used as antioxidants.

The pharmaceutical composition can also comprise one or more glidants to improve the flowing properties of the formulation. For example, talc or silica in colloidal anhydrous form can be used.

The composition can comprise a lubricant selected from, but not limited to the group consisting of stearic acid, magnesium stearate, calcium stearate, hydrogenated vegetable oil, hydrogenated castor oil, sodium stearyl fumarate, talc, macrogols and mixtures thereof. It is preferred that the additional excipients include at least one lubricant selected from hydrogenated castor oil, talc and magnesium stearate.

The composition according to the invention is present in a solid dosage form, preferably in a tablet form and more preferably in the form of film-coated tablet. The film-coating can be an enteric coating, a modified-release coating, a protective coating, a taste-masking coating and the like.

A preferred film-coating comprises a film-forming polymer and optionally plasticizer, antiadhesive, colouring agents and/or other ingredients.

Examples of suitable film-forming polymers are selected from, but not limited to the group consisting of cellulose derivatives, such as methylcellulose, hydroxypropyl methylcellulose, hydroxypropylcellulose, ethylcellulose, cellulose acetate, cellulose acetate phthalate, sodium carboxymethylcellulose and calcium carboxymethylcellulose, polyvinyl alcohol, polyvinyl acetate, polyvinyl acetate phthalate, methacrylic acid polymers, polyethylene glycol, polyvinylpirrolidone, gelatin, shellac and mixtures thereof. Waxes can also be used as film-forming polymers, like carnauba wax or microcrystalline wax. Preferably, cellulose derivatives are used as film-forming polymers.

Examples of suitable plasticizers include, but are not limited to polyols, such as glycerol, propylene glycol and polyethylene glycols, organic esters, such as phtalate esters, dibutyl sebacate, citrate esters and triacetin, and oils/glycerides, such as castor oil, acetylated monoglycerides and fractionated coconut oil.

Talc can be used as an antiadhesive.

A composition is particularly preferred which comprises
(a) 1 to 50 %, preferably 1 to 40 %, more preferably 1 to 30 % by weight of donepezil hydrochloride,
(b) 1 to 90 %, preferably 20 to 85%, more preferably 25 to 35 % by weight of a cellulose and/or starch derivative, and optionally
(c) 1 to 90 %, preferably 10 to 40% by weight of at least one additional pharmaceutically acceptable excipient such as lactose monohydrate and/or microcrystalline cellulose.

The invention also relates to a process for preparing the composition according to the invention comprising
(i) converting components (a) and (b) and optionally other excipients into a particulate form and
(ii) optionally coating the composition obtained in step (i).

In step (i) components (a) and (b) and optionally other excipients are converted into a particulate form. This can be done by any known process such as wet granulation, dry granulation (slugging or roller compaction) or direct compression. The method of preparation and the type of excipients are selected to allow rapid compression process and give the tablet formulation the desired physical characteristics.

Wet granulation is used to convert a powder mixture into granules having suitable flow and cohesive properties for compression. Typically, wet granulation comprises the following steps:
a) mixing of the drug with at least one excipient in a suitable blender,
b) spraying of the granulation fluid onto the powder mixture,
c) optionally screening of the wet granules,
d) drying of the wet granules and screening.

The granulation fluid can, for example, be water, organic solvents or mixtures thereof, solutions of suitable binders in water, organic solvents or mixtures thereof and the like. Most preferably, water or aqueous binder solutions are used as granulation liquid.

For the wet granulation process state of the art granulators can be used, for example high shear granulator, low shear granulator or fluid bed granulator.

Dry granulation can also be used to convert powders into granules. Typically, dry granulation comprises the following steps:
a) mixing of the drug with at least one excipient in a suitable blender,
b) slugging or roller compaction of the powder mixture,
c) dry screening.

The compaction step is preferably carried out on roller compactor. A compaction force of between 15 and 65 kN is preferably used.

Direct compression can also be used to convert the starting materials (a), (b) and additional other excipients into a particulate form.

Thus, the invention particularly relates to a direct compression process for preparing the composition according to the invention comprising
(i') mixing of (a) a drug selected from donepezil or a pharmaceutically acceptable salt or solvate thereof and (b) at least one matrix former and optionally further excipients to obtain a compression mixture,
(i") compressing the mixture obtained in step (i') to form a solid pharmaceutical composition, and
(ii) optionally coating the composition obtained in step (i").

The compression of the granulates (either prepared by wet or dry granulation) or of the compression mixture to tablet cores can be carried out in a conventional tabletting machine, e.g. in an eccentric tabletting machine or a rotary compression machine, preferably at a compression force greater than 2 kN. The tablet cores may vary in shape and be, for example, round, oval, oblong, cylindrical or any other suitable shape, and may also vary in size depending on the concentration of the therapeutic agents. In step (ii) the composition obtained in step (i) can be coated with a coating material. Tablet coating equipment known from the state of the art can be used for this step. Water, organic solvents such as ethanol, acetone, isopropanole or mixtures thereof can be used for preparing coating dispersions.

The invention also relates to a process for preparing a composition according to the invention comprising
(a) granulating (i) a drug selected from donepezil or a pharmaceutically acceptable salt or solvate thereof, (ii) at least one matrix former and optionally further excipients to obtain granules,
(b) mixing the granules obtained in step (a) with extragranular excipients to obtain a mixture,
(c) compressing the mixture obtained in step (b) to form a solid pharmaceutical composition, and
(d) optionally coating the solid pharmaceutical composition obtained in step (c) with a coating material.

In step (a), components (i) and (ii) and optionally other excipients are converted into granules. This can be done by any known process such as wet or dry granulation. It is preferred that step (a) is performed by wet granulation. More preferably, wet granulation is performed using water or aqueous binder solutions. For example, wet granulation can be carried out in a high shear mixer. Prior to granulation step (a), the components to be converted into granules, e.g. drug (i), matrix former (ii) and optionally other excipients, can be thoroughly mixed to ensure a high homogeneity of the obtained granules.

At the end of step (a), the obtained granules are optionally dried, e.g. by using a fluid bed drier or any other drying technique like tray drying or vacuum drying, and sieved, e.g. by using a 1.0 mm sieve, thus obtaining granules having a d90 less than 1.0 mm, preferably less than 800 µm. The particle size can be determined by laser diffraction or sieve analysis.

In step (b), the granules obtained in step (a) are mixed with other excipients such as lubricants and glidants. Mixing (b) can be carried out using a biconic mixer, a high shear mixer or any other suitable mixer.

In step (c), the mixture obtained in step (b) is compressed to obtain a solid pharmaceutical composition which is preferably in the form of a tablet. Tablet compression equipment known from the state of the art can be used for this step, such as a high speed rotary machine. In a preferred embodiment, the mixture of step (b) is compressed using a compression pressure of about 2 to about 30 kN, preferably less than 15 kN. Preferably, the obtained tablet has a diameter from 5 to 15 mm, preferably 6 to 12 mm, more preferably 7 to 9 mm and a weight of 40 to 800 mg, preferably 80 to 600 mg and more preferably 150 to 480 mg. The shape of tablets can be of any known form, oblong and round shape being preferred in order to alleviate the swallowing of the tablet by patients having problems with swallowing, especially geriatric patients.

In step (d) the composition obtained in step (c) can be coated with a coating material. Tablet coating equipment known from the state of the art can be used for this step. Water, organic solvents such as ethanol, acetone, isopropanol or mixtures thereof can be used for preparing coating dispersions.

The invention is also directed to pharmaceutical compositions obtainable by the above processes according to the invention.

The stability of the solid pharmaceutical compositions according to the invention can be further improved by packing them into primary packaging having decreased permeability for water vapour such as high density polyethylene (HDPE) containers with closure, such as polypropylene (PP) closure and with or without a desiccant; aluminium foil blisters or polychlorotrifluoroethylene (Aclar®) blisters or any other suitable water vapour low-permeable packaging such as blisters made of multilayer polymeric foil where different polymeric materials are combined into a single foil. Additionally inert gases such as nitrogen, argon or helium; or vacuum can be used during the manufacturing of the solid pharmaceutical composition according to the first and second aspect of the invention as well as during their packaging to assure inert atmosphere around the solid oral dosage form, such as tablet or capsule, in the sealed primary packaging. Inert atmosphere according to present invention means that the concentration of oxygen in the atmosphere around the solid dosage form, packed in the primary packaging is less than 10 vol%, preferably less than 5 vol% and most preferably less than 2 vol%. The concentration of oxygen in the atmosphere surrounding the tablet can be determined by gas chromatography.

The invention is further illustrated by reference to the following examples which are not intended to limit the scope of protection.

### Examples

### Reference Example R1 - Formulation as per WO2006/070930:

| Ingredient | Amount per tablet (mg) |
|---|---|
| Donepezil HCl | 23.00 |
| Ethylcellulose | 44.00 |
| Eudragit L 100-55 | 42.00 |
| Lactose monohydrate | 84.40 |
| Hydroxypropylcellulose | 6.00 |
| Magnesium stearate | 0.60 |
| **Total** | **200.00** |

Donepezil HCl, ethylcellulose, Eudragit L 100-55 and lactose monohydrate were mixed and granulated with a solution of hydroxypropylcellulose in water. After drying and sieving, magnesium stearate was added and the obtained mixture was compressed into tablets using round punches.

### Reference Example R2 - Formulation as per WO2006/070930 without acidic polymer:

| Ingredient | Amount per tablet (mg) |
|---|---|
| Donepezil HCl | 23.00 |
| Ethylcellulose | 50.00 |
| Lactose monohydrate | 120.40 |
| Hydroxypropylcellulose | 6.00 |
| Magnesium stearate | 0.60 |
| **Total** | **200.00** |

Donepezil HCl, ethylcellulose and lactose monohydrate were mixed and granulated with a solution of hydroxypropylcellulose in water. After drying and sieving, magnesium stearate was added and the obtained mixture was compressed into tablets using round punches.

### Reference Example R3 - Formulation as per WO2006/070930 with addition of citric acid

| Ingredient | Amount per tablet (mg) |
|---|---|
| Donepezil HCl | 23.00 |
| Ethylcellulose | 50.00 |
| Eudragit L 100-55 | 36.00 |
| Citric acid | 2.50 |
| Lactose monohydrate | 82.40 |
| Hydroxypropylcellulose | 6.00 |
| Magnesium stearate | 0.60 |
| **Total** | **200.00** |

Donepezil HCl, ethylcellulose, Eudragit L 100-55 and lactose monohydrate were mixed and granulated with a solution of hydroxypropylcellulose and citric acid in water. After drying and sieving, magnesium stearate is added and the obtained mixture is compressed into tablets using round punches.

### Example 1:

| Ingredient | Amount per tablet (mg) |
|---|---|
| Donepezil HCl | 23.00 |
| Lactose monohydrate | 113.00 |
| Hydroxypropyl methylcellulose (Methocel K4M) | 60.00 |
| Magnesium stearate | 4.00 |
| **Total** | **200.00** |

### Example 2:

| Ingredient | Amount per tablet (mg) |
|---|---|
| Donepezil HCl | 23.00 |
| Lactose monohydrate | 113.00 |
| Hydroxypropyl methylcellulose (Methocel K4M) | 40.00 |
| Hydroxypropyl methylcellulose (Methocel K100 LV) | 20.00 |
| Magnesium stearate | 4.00 |
| **Total** | **200.00** |

### Example 3:

| Ingredient | Amount per tablet (mg) |
|---|---|
| Donepezil HCl | 23.00 |
| Hydroxypropyl methylcellulose (HPMC K100M) | 150.00 |
| Lactose monohydrate | 50.00 |
| Microcrystalline cellulose | 224.00 |
| Magnesium stearate | 3.00 |
| **Total** | **450.00** |

The compositions according to Examples 1 to 3 were prepared by mixing all ingredients using a biconic mixer and compressing the obtained mixture into tablets using round punches. Donepezil HCl of polymorphic form I was used.

### Example 4:

| Ingredient | Amount per tablet (mg) |
|---|---|
| Donepezil HCl | 23.00 |
| Hydroxypropyl methylcellulose (HPMC K100M) | 120.00 |
| Lactose monohydrate | 158.00 |
| Purified water | q.s. |
| Carbomer (Carbopol 71G NF) | 20.00 |
| Hydroxypropyl methylcellulose (HPMC K100M) | 120.00 |
| Magnesium stearate | 3.00 |
| **Total** | **450.00** |

Donepezil HCl, the first portion of hydroxypropyl methylcellulose (HPMC K100M) and lactose monohydrate were mixed and granulated with purified water. Carbomer (Carbopol 71G NF) and the other portion of hydroxypropyl methylcellulose (HPMC K100M) were admixed. After addition of magnesium stearate tablets were compressed using round punches.

### Example 5:

| Ingredient | Amount per tablet (mg) |
|---|---|
| Donepezil HCl | 23.00 |
| Hydroxypropyl methylcellulose (HPMC K100 LV) | 60.00 |
| Sorbitol | 20.00 |
| Microcrystalline cellulose | 84.00 |
| PEG 6000 | 10.00 |
| Talc | 3.00 |
| **Total** | **200.00** |

### Example 6:

| Ingredient | Amount per tablet (mg) |
|---|---|
| Donepezil HCl | 23.00 |
| Pregelatinized starch (Swelstar MX-1) | 60.00 |
| Sorbitol | 20.00 |
| Microcrystalline cellulose | 84.00 |
| PEG 6000 | 10.00 |
| Talc | 3.00 |
| **Total** | **200.00** |

The compositions according to Examples 5 and 6 were prepared by mixing all ingredients and compressing the obtained mixture into tablets using round punches.

### Example 7:

| Ingredient | Amount per tablet (mg) |
|---|---|
| Donepezil HCl | 23.00 |
| Hydroxypropyl methylcellulose (HPMC K100M) | 120.00 |
| Lactose monohydrate | 158.00 |
| Purified water | q.s. |
| Hydroxypropyl methylcellulose (HPMC K100M) | 120.00 |
| Magnesium stearate | 9.00 |
| **Total** | **430.00** |

Donepezil HCl, a first portion of hydroxypropyl methylcellulose (HPMC K100M) and lactose monohydrate were mixed and granulated with purified water. The other portion of hydroxypropyl methylcellulose (HPMC K100M) was added. After addition of magnesium stearate tablets were compressed using round punches.

### Example 8:

| Ingredient | Amount per tablet (mg) |
|---|---|
| Donepezil HCl | 23.00 |
| Hydroxypropyl methylcellulose (HPMC K4M) | 275.00 |
| Calcium phosphate anhydrous | 73.50 |
| Lactose monohydrate | 73.50 |
| Magnesium stearate | 5.00 |
| **Total** | **450.00** |

### Example 9:

| Ingredient | Amount per tablet (mg) |
|---|---|
| Donepezil HCl | 23.00 |
| Eudragit RS PO | 80.00 |
| Hydroxypropyl methylcellulose (HPMC K4M) | 233.00 |
| Carbomer (Carbopol 971P NF) | 40.00 |
| Hydroxypropyl cellulose (Klucel EF) | 12.00 |
| Talc | 8.00 |
| Magnesium stearate | 4.00 |
| **Total** | **400.00** |

The compositions according to Examples 8 and 9 were prepared by mixing all ingredients and compressing the obtained mixture into tablets using round punches.

### Example 10:

| Ingredient | Amount per tablet (mg) |
|---|---|
| Donepezil HCl | 23.00 |
| Hydroxypropyl methylcellulose (HPMC K100M) | 60.00 |
| Lactose monohydrate | 43.00 |
| Purified water | q.s. |
| Carbomer (Carbopol 71G NF) | 10.00 |
| Hydroxypropyl methylcellulose (HPMC K100M) | 60.00 |
| Magnesium stearate | 4.00 |
| **Total** | **200.00** |

Donepezil HCl, the first portion of hydroxypropyl methylcellulose (HPMC K100M) and lactose monohydrate were mixed and granulated with purified water. Carbomer (Carbopol 71G NF), hydroxypropyl methylcellulose (HPMC K100M) were mixed. After addition of magnesium stearate mixture was compressed into tablets.

The tablets of Examples R1, R2, R3, 4, 7 and 8 were packed in glass vials and stored at 60°C and 10% relative humidity for 21 days. The related substances were determined with HPLC, using a Gemini C18, 150 mm x 4.6 mm i.d., 3 µm particles.

The chromatographic conditions of the HPLC analysis were as follows:
*Mobile phase:*
   A: 0.1% perchloric acid solution with pH 3.2 (Adjusted with 10% TEA solution)
   B: acetonitrile

| Time (min) | 0 | 10 | 20 | 25 | 25.1 | 30 |
|---|---|---|---|---|---|---|
| % A | 70 | 70 | 20 | 20 | 70 | 70 |
| % B | 30 | 30 | 80 | 80 | 30 | 30 |

The mobile phase was filtered through a 0.45 µm membrane filter and degassed prior to use.

*Flow rate:* about 0.7 ml/min.

*Detection:* UV, wavelenght 230nm.

### Amount of impurities determined by HPLC (stress condition: 60°C - 21 days) :

| Sample | Related substances | |
|---|---|---|
| | Impurity N* (%) | Total (%) |
| Example R1 | 0.06 | 0.06 |
| Example R2 | 0.07 | 0.07 |
| Example R3 | 0.09 | 0.40 |
| Example 4 | b.r.l. | b.r.l. |
| Example 7 | b.r.l. | b.r.l. |
| Example 8 | b.r.l. | b.r.l. |

| | | |
|---|---|---|
| *Chemical name of impurity N: 1-Benzyl-4-[(5,6-dimethoxy-1-indanon-2-yl)methyl]piperidine 1-oxide b.r.l. = below reporting level, i.e. ≤ 0.05 % | | |

As can be seen from the above table, the tablets according to the present invention shows an excellent stability compared to the tablets according to the reference examples.

## Claims

1. Solid pharmaceutical composition comprising
(a) at least one drug selected from donepezil or a pharmaceutically acceptable salt or solvate thereof, and
(b) at least one matrix former which is selected from the group consisting of cellulose derivatives, starch derivatives and mixtures thereof, wherein the matrix former has a viscosity of 80 to 120,000 mPa·s, determined for a 2 % (w/v) aqueous solution at 20°C.

2. Composition according to claim 1, wherein the cellulose derivative (b) is a cellulose ether and/or a cellulose ester.

3. Composition according to claim 2, wherein the cellulose derivative (b) is a cellulose polymer in which the hydroxyl groups of cellulose are at least partially replaced by substituents -OR with R being selected from the group consisting of alkyl, hydroxyalkyl, -C(O)alkyl, -C(O)aryl and mixtures thereof, wherein said alkyl or aryl can be unsubstituted or optionally substituted.

4. Composition according to claim 3, wherein the cellulose derivative (b) is selected from the group consisting of ethylcellulose, methylcellulose, cellulose acetate, cellulose acetate phthalate, hydroxymethyl cellulose, hydroxyethyl cellulose, hydroxypropyl cellulose, hydroxypropyl methylcellulose and mixtures thereof

5. Composition according to claim 3 or 4, wherein the cellulose derivative (b) has a hydroxyalkoxy content of 0 to 80 % and/or an alkoxy content of 0 to 40 %.

6. Composition according to any one of claims 1 to 5, wherein the cellulose derivative (b) is hydroxypropyl methylcellulose.

7. Composition according to claim 1, wherein the starch derivative (b) is pregelatinized starch.

8. Composition according to any one of claims 1 to 7, wherein the matrix former (b) has a viscosity of 80 to 120 mPa·s or a viscosity of 3,000 to 5,600 mPa·s or a viscosity of 11,250 to 21,000 mPa·s or a viscosity of 80,000 to 120,000 mPa·s, determined for a 2 % (w/v) aqueous solution at 20°C.

9. Composition according to any one of claims 1 to 8, wherein the matrix former (b) has a bulk density of less than 0.5 g/cm³.

10. Composition according to any one of claims 1 to 9, wherein the matrix former (b) has an average particle size of about 50 µm to about 200 µm.

11. Composition according to any one of claims 1 to 10, wherein the composition comprises an additional matrix former (c), selected from the group consisting of a cross-linked acrylic acid polymer and mixtures thereof.

12. Composition according to any one of claims 1 to 11, wherein the composition further comprises at least one other excipient selected from the group consisting of diluents, gel strength regulators, binders, stabilizers, glidants, lubricants, colorants and mixtures thereof.

13. Composition according to any one of claims 1 to 12, wherein the composition is **characterized by** an *in vitro* release profile wherein on average about 15 to about 55 % of drug (a) is dissolved within 2 hours, about 40 to about 80 % of drug (a) is dissolved within 6 hours and more than 80 % of drug (a) are dissolved within 20 hours after placement of the composition in a standard dissolution test using Apparatus 1 at a rotation speed of 100 rpm and a dissolution medium of 0.1 M HCl, pH 6.8 at 37 ± 0.5°C

14. Process for preparing a solid pharmaceutical composition according to any one of claims 1 to 13 comprising
(i) converting components (a) and (b) and optionally other excipients into a particulate form and
(ii) optionally coating the composition obtained in step (i).

15. Process according to claim 14, wherein step (i) is carried out by direct compression comprising
(i') mixing of (a) a a drug selected from donepezil or a pharmaceutically acceptable salt or solvate thereof and (b) at least one matrix former and optionally other excipients to obtain a compression mixture, and
(i") compressing the mixture obtained in step (i') to form a solid pharmaceutical composition.
